# EUROPEAN PATENT APPLICATION

(11) **EP 4 799 616 A1**
(43) Date of publication of application: **02.09.2026**
(21) Application number: 26161218.8
(22) Date of filing: 27.02.2026
(51) Int. Cl.: A61K 31/194, A61K 31/197, A61K 31/198, A61K 31/375, A61K 31/455, A61K 9/00, A61K 31/51, A61K 31/525, A61K 31/122, A61K 31/185, A61K 31/19, A23L 33/15, A61P 9/00, A61P 9/06, A61P 25/24, A61P 25/28

(54) **A MICRONUTRIENT PHARMACEUTICAL COMPOSITION FOR BIOENERGY STIMULATION FOR MAMMAL**

(30) Priority: 27.02.2025 US 202563764500 P
(71) Applicant: Nass, Boukelien Yentl Sundari, 6241NA Bunde (NL); Rath, Anais Madeleine Ilyse, 6241Na Bunde (NL); Rath, Mael, 6241NA Bunde (NL)
(72) Inventor: Niedzwiecki, Aleksandra, Henderson, 89044 (US); Rath, Matthias W., deceased (US); Goc, Anna, San Jose, 95133 (US)
(74) Representative: Altmann Stößel Dick Patentanwälte PartG mbB

(57) **Abstract**

A micronutrient composition comprising several combinations of individual ingredients in forming Mix 11, G12, G13 and core mix formulation. The Core mix contains magnesium citrate, alpha-Ketoglutaric acid, L-Citrulline and Taurine. The Mix 11, G12 and G13 contains Magnesium Citrate, Alpha-Ketoglutaric Acid, Niacinamide, L-Glutamine, L-Citrulline, Sodium Pyruvate, Succinic acid, Vitamin B complex, Taurine, L-Cysteine, L-Methionine, L-Ascorbic Acid, Coenzyme Q10 or combination thereof. A method of administering and treating a specific disease which is due to one of an oxidative stress, impaired mitobiogenesis and ATP production in a mammal is disclosed.

## Description

### FIELD OF STUDY

The instant study is focused on a micronutrient pharmaceutical composition that protects, improves and enhances bioenergy in a cell for mammal.

### BACKGROUND

Deficiency of or inadequate bioenergy production results in cellular malfunctions causing many negative effects on physical and mental development, performance and the function of all organs. Impaired bioenergy supply has been linked to a wide spectrum of diseases classified as mitochondrial diseases including arrhythmia, heart failure and other cardiovascular conditions as well as depression, mental decline, dementia and many other health conditions.

Since health issues related to impaired bioenergy affect the lives of hundreds of millions of people worldwide and are a major cause of rising healthcare costs there is an urgent need for the development of enhancers of mitochondrial biological energy at the cellular level. Conventional medicine did not develop so far, specific pharmaceutical drugs or other approaches to enhance the energy carriers and improve bioenergy inside the body.

The Krebs cycle, also known as the Citric Acid or Tricarboxylic acid cycle, is a vital biochemical bioenergy pathway located in mitochondria. By a sequence of reactions involving electron transfers, the Krebs cycle generates most of the energy for our bodies utilizing carbohydrates and fats from food as biological fuel for cellular metabolism. Various enzymes and cofactors are involved in the respiratory cycle to produce Adenosine triphosphate, also known as ATP, which is the main energy carrier for all living things. ATP is generated from Adenosine diphosphate (ADP) and converted back to ADP after releasing stored energy- thus creating the ATP-ADP cycle.

Cells adapt to increased energy requirements (such as induced by exercise or environmental factors) by increasing the number of cellular energy power plants -the mitochondria.

Mitochondria are not created de novo, but originate from our mothers, however cells can increase their number to meet metabolic energy demands as well as slowing down age-related damage. Mitochondrial biogenesis can become a therapeutic target for various diseases, including neurodegenerative disorders and metabolic syndrome which is associated with increased risk of heart disease, stroke and type2 diabetes.

There is a need to address these cycles when cell is under stress due to infection or any other reason.

### SUMMARY

In the instant disclosure various combinations of micronutrients as pharmaceutical micronutrient composition (Mix 11, G12 and G13) are administered and used to enhance, cure or manage efficient functioning of each step of the metabolic pathway for disease prevention, faster recovery from injury and overall wellness. The intent of this disclosure is to describe how to develop a mix of ingredients which help protect cell viability and stimulate mitochondria formation and bioenergy production. These parameters such as stimulation of mitochondrial formation and bioenergy production are important for quick recovery from injury, for protection from harmful agents common in our environment and for overall quality of life.

Any references to methods of treatment of a mammal by administering a pharmaceutical composition described herein are likewise to be interpreted as references to the respective pharmaceutical compositions as such as well as to the respective pharmaceutical compositions for use in treating a mammal.

In another embodiment, effects of individual components present in all the mixes and combined ingredients as different mixes (Mix 11, G12 and G13) on ATP production, effects on ATP synthesis, mitobiogenesis in different types of human cells, human cardiomyocytes (AC16), human skeletal muscle cells (differentiated), mouse immortalized microglial cells, human liver cells (HepG2) and rat cardiomyoblast cells (H9c2) are illustrated and the best pharmaceutical composition is chosen for treating a mammal to recover from injury, protection from harmful agents common in our environment and for overall quality of life.

In one embodiment a physiological dose for a mammal was calculated based on daily consumption. The formula was packaged in drug formulation for easy consumption. In one embodiment, the Mixes at least one ingredient selected from the group consisting of Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine, L-Cysteine and combination thereof. In one embodiment, a deletion or addition of other ingredients are disclosed.

In one embodiment, Mix 11 comprises at least one ingredient selected from the group consisting of Magnesium Citrate, Alpha-Ketoglutaric Acid, Niacinamide, L Glutamine, L Citrulline, Sodium Pyruvate, Succinic acid, Vitamin B Complex, Taurine, L-Methionine, L- Cysteine and combination thereof. In another embodiment, Mix 11 comprises Magnesium Citrate, Alpha-Ketoglutaric Acid, Niacinamide, L Glutamine, L Citrulline, Sodium Pyruvate, Succinic acid, Vitamin B Complex, Taurine, L-Methionine, and L- CysteineIn another embodiment, Mix 11 consists of Magnesium Citrate, Alpha-Ketoglutaric Acid, Niacinamide, L Glutamine, L Citrulline, Sodium Pyruvate, Succinic acid, Vitamin B Complex, Taurine, L-Methionine, and L-Cysteine.

In one embodiment Core mix comprises at least one ingredient selected from the group consisting of s Magnesium Citrate, Alpha-Ketoglutaric Acid, L Citrulline and Taurine.

In one embodiment Mix G 12 comprises at least one ingredient selected from the group consisting of Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, L-Cysteine and combination thereof. In another embodiment, Mix G 12 comprises Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, and L- Cysteine. In another embodiment, Mix G 12 consists of Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, and L- Cysteine.

In one embodiment, G 13 comprises at least one ingredient selected from the group consisting of Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine, L- Cysteine and combination thereof. In one embodiment, a deletion or addition of other ingredients are done to increase the efficacy of the G 13. In one embodiment, G 13 comprises Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine and L- Cysteine. In one embodiment, G 13 consists of Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine and L- Cysteine.

In one embodiment, the range concentration for each ingredients are Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000mg , Coenzyme Q10 in the range of 1-3,000mg, L Glutamine in the range of 10-50,000 mg, L Citrulline in the range of 10-20,000mg, Sodium Pyruvate in the range of 10-50,000mg, Succinic acid in the range of 10-30,000mg, Vitamin B Complex contains B1 in the range of 2.4 mg-48 mg, B2 in the range of 3.7 mg- 74mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, Biotin in the range of 110 mcg-2200 mcg, Taurine in the range of 10-10,000 mg, L-Methionine in the range of 10-20,000 mg, L- Cysteine in the range of 10-20,000 mg.

In one embodiment, G 13 mix comprises Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000mg , Coenzyme Q10 in the range of 1-3,000mg, L Glutamine in the range of 10-50,000 mg, L Citrulline in the range of 10-20,000mg, Sodium Pyruvate in the range of 10-50,000mg, Succinic acid in the range of 10-30,000mg, Vitamin B Complex contains B1 in the range of 2.4 mg-48 mg, B2 in the range of 3.7 mg- 74mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, Biotin in the range of 110 mcg-2200 mcg, Taurine in the range of 10-10,000 mg, L-Methionine in the range of 10-20,000 mg, L- Cysteine in the range of 10-20,000 mg. In one embodiment, G 13 mix consists of Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000mg , Coenzyme Q10 in the range of 1-3,000mg, L Glutamine in the range of 10-50,000 mg, L Citrulline in the range of 10-20,000mg, Sodium Pyruvate in the range of 10-50,000mg, Succinic acid in the range of 10-30,000mg, Vitamin B Complex contains B1 in the range of 2.4 mg-48 mg, B2 in the range of 3.7 mg-74mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, Biotin in the range of 110 mcg-2200 mcg, Taurine in the range of 10-10,000 mg, L-Methionine in the range of 10-20,000 mg, L- Cysteine in the range of 10-20,000 mg.

In one embodiment, a method of administering the G 13 mix comprising Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000mg , Coenzyme Q10 in the range of 1-3,000mg, L Glutamine in the range of 10-50,000 mg, L Citrulline in the range of 10-20,000mg, Sodium Pyruvate in the range of 10-50,000mg, Succinic acid in the range of 10-30,000mg, Vitamin B Complex contains B1 in the range of 2.4 mg-48 mg, B2 in the range of 3.7 mg- 74mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, Biotin in the range of 110 mcg-2200 mcg, Taurine in the range of 10-10,000 mg, L-Methionine in the range of 10-20,000 mg, L- Cysteine in the range of 10-20,000 mg. In a further embodiment, a method of administering the G 13 mix consisting of Magnesium Citrate in the range of 10-30,000 mg, Alpha-Ketoglutaric Acid in the range of 10-30,000 mg, Niacinamide in the range of 1-10,000 mg, Vitamin C as L- Ascorbic acid in the range of 5-50,000mg , Coenzyme Q10 in the range of 1-3,000mg, L Glutamine in the range of 10-50,000 mg, L Citrulline in the range of 10-20,000mg, Sodium Pyruvate in the range of 10-50,000mg, Succinic acid in the range of 10-30,000mg, Vitamin B Complex contains B1 in the range of 2.4 mg-48 mg, B2 in the range of 3.7 mg- 74mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, Biotin in the range of 110 mcg-2200 mcg, Taurine in the range of 10-10,000 mg, L-Methionine in the range of 10-20,000 mg, L- Cysteine in the range of 10-20,000 mg. Finally, the present invention is described further in the detailed description to further illustrate various aspects of the present invention.

### BRIEF DESCRIPTION OF DRAWINGS

Example embodiments are illustrated by way of example and not limited in the figures of the accompanying drawings, in which like references indicate similar elements and in which:
**Figure 1** shows human myocyte cells were grown to confluency and treated with test ingredients for 24 hours and ATP were measured.
**Figure 2** shows rat cardiac myoblasts cells were grown to confluency and treated with test ingredients for 24 hours and was used to quantify mitochondrial DNA coded protein.
**Figure 3** shows microglial cells were exposed to test compounds for 24 hours following by Hydrogen Peroxide their viability was evaluated.
**Figure 4** shows effect of individual test ingredients on ATP synthesis in human cardiomyocytes cells.
**Figure 5** shows effect of individual test ingredients on ATP synthesis in human skeletal muscle cells (differentiated).
**Figure 6** shows effect of individual test ingredients on ATP synthesis in mouse microglial cells.
**Figure 7** shows effect of individual test ingredients on ATP synthesis in human liver cells (HepG2).
**Figure 8** shows effect of individual test ingredients on ATP synthesis in rat cardio-myoblasts (H9c2).
**Figure 9A** and **Figure 9B** show the effect of individual test ingredients on mitochondria formation in human cardiac myocytes (AC16).
**Figure 10** shows Changes in ratios of Cox-I to SDH-A in human cardiomyocytes (AC16) exposed to individual nutrients.
**Figure 11a** and **Figure 11b** show the effects of individual compounds on mitochondria formation in differentiated human skeletal muscle cells evaluated by mitochondria encoded COX-I protein and nuclear encoded protein SDH-A.
**Figure 12** shows changes in ratios of Cox-I to SDH-A in human differentiated skeletal muscle cells exposed to individual nutrients.
**Figure 13A** and **Figure 13B** shows the effects of individual compounds on mitochondria formation in microglial cells by evaluating mitochondrial encoded protein COX-I and nuclear encoded SDH-A.
**Figure 14** shows changes in ratios of Cox-I to SDH-A in mouse immortalized microglial cells exposed to individual nutrients.
**Figure 15A** and **Figure 15B** shows the effects of individual compounds on mitochondria formation in human liver cells (HepG2) evaluated by mitochondria encoded COX-I protein and nuclear encoded protein SDH-A.
**Figure 16** shows changes in ratios of Cox-I to SDH-A in human liver cells HepG2 exposed to individual nutrients.
**Figure 17A** and **Figure 17B** shows the effects of test ingredients on mitochondrial DNA coded protein COX-I and nuclear encoded SDH-A in rat cardiac myoblasts (H9c2).
**Figure 18** shows changes in ratios of Cox-I to SDH-A in rat cardiomyoblasts (H9c2) exposed to individual nutrients.
**Figure 19A** and **Figure 19B** shows the effects of nutrient compositions M11, G12, G13 and Core on ATP synthesis in human cardiomyocytes and differentiated human skeletal muscle cells.
**Figure 20A** and **Figure 20B** shows the effects of nutrient compositions M11, G12, G13 and Core on ATP synthesis in mouse immortalized microglial cells and human liver HepG2 cells.
**Figure 21** shows the effects of nutrient compositions M11 and Core on ATP synthesis in rat cardiomyoblasts (H9c2).
**Figure 22A** and **Figure 22B** shows the effects of M11, G12 and G13 compared to control on mitobiogenesis expressed as ratio of COX-I (mitochondria encoded gene) to SDH-A (nucleus encoded) in human cardiomyocytes and differentiated human skeletal muscle cells.
**Figure 23A** and **Figure 23B** shows the effects of Mix (G13) and Core compared to control on mitobiogenesis expressed as ratio of COX-I (mitochondria encoded gene) to SDH-A (nucleus encoded) in human liver cells (HepG2) and mouse microglial cells.
**Figure 24** shows the effects of Mix 11 and Core compared to control on mitobiogenesis expressed as ratio of COX-I (mitochondria encoded gene) to SDH-A (nucleus encoded) in rat cardiomyoblasts (H9c2).
**Figure 25A, Figure 25B****,** **Figure 25** **C** and **Figure 25D** shows the effects of individual compounds and their combinations on quenching of cellular reactive species (ROS) in four different cell lines.
**Figure 26** shows the effects of individual natural compounds and their defined combinations in Mix 11 and Core on viability of mouse immortalized microglial cells.
**Figure 27** shows the effects of individual natural compounds and their defined combinations in Mix 11 and Core on viability of mouse immortalized microglial cells

Other features of the present embodiments will be apparent from the accompanying drawings and from the detailed description that follows.

### DETAILED DESCRIPTION

Mitochondrial energy generation comprises several tri-carboxylic acids mediators (Krebs cycle) and the electron transfer chain (respiratory chain) leading to generation of ATP molecules. Mitochondrial biogenesis involves coordinated expression of both mitochondrial and nuclear genomes, in the respiratory chain, the succinate dehydrogenase (Complex II or SDH), is the product of four nuclear-encoded genes. This protein complex links the tricarboxylic acid cycle with the electron transport chain. SDH is composed of four subunits that must translocate independently to the mitochondria and assemble into a mature protein complex embedded in the inner mitochondrial membrane. Another part of the electron transport chain in mitochondria essential for cellular respiration is cytochrome c oxidase (Complex IV), which is responsible for transferring electrons from cytochrome c to oxygen, ultimately powering ATP synthesis mitochondrial complex and producing water. Crucial component for cytochrome c oxidase subunit I (also known as MT-CO1) is COX1 protein. This protein is coded by COX1 gene located on mitochondrial DNA in eukaryotes. The instant disclosure shows various combinations of individual components of pharmaceutical composition, and their effect on preventing and/or treating disease that originate from oxidative stress, impaired mitobiogenesis and ATP production, and are used as a pharmaceutical composition and administered to a human suffering from mitochondrial deficiency or dysfunction related to specific diseases.

Materials and Methods: Cell Lines: Rat Cardiomyoblasts (H9c2) were obtained from ATCC (Virginia, USA). It is derived from embryonic BD1X rat heart tissue that exhibits many of the properties of skeletal muscle. Cells were maintained in Dulbecco's Modified Eagle's Medium (DMEM) from Thermofisher (MA, USA) supplemented with 10% Fetal Bovine Serum (FBS) and 1% Penicillin-Streptomycin (PS) from Millipore, Sigma (MA, USA).

Microglial Cells (IMG) were obtained from Kerafast. It is an immortalized microglial cell line isolated from the brains of adult mice. Cells were maintained in DMEM supplemented with 10% FBS and 1% PS. from Millipore, Sigma (MA, USA).

Human myocytes (AC10) were obtained from ATCC (Virginia, USA). It is derived from adult ventricular tissue. Cells were maintained in Dulbecco's Modified Eagle's Medium F12 (DMEM F12) from Thermofisher (MA, USA) supplemented with 10% Fetal Bovine Serum (FBS) and 1% Penicillin-Streptomycin (PS) from Millipore, Sigma (MA, USA).

Cell Lines: Human HepG2 cells were obtained from ATCC (Virginia, USA). Cells were maintained in Dulbecco's Modified Eagle's Medium (DMEM) from Thermofisher (Massachusetts, USA) supplemented with 10% Fetal Bovine Serum (FBS) and 1% Penicillin-Streptomycin (PS) from Millipore, Sigma (Massachusetts, USA).

Microglial Cells (IMG) were obtained from Kerafast (California, USA). It is an immortalized microglial cell line isolated from the brains of adult mice. Cells were maintained in DMEM supplemented with 10% Fetal Bovine Serum (FBS) and 1% Penicillin-Streptomycin (PS) from Millipore, Sigma (Massachusetts, USA).

Cardiac myocytes (AC16) were obtained from ATCC (Virginia, USA). This cell line is derived from human adult ventricular tissue. Cells were maintained in Dulbecco's Modified Eagle's Medium F12 (DMEM F12) from Thermofisher (Massachusetts, USA) supplemented with 10% Fetal Bovine Serum (FBS) and 1% Penicillin-Streptomycin (PS) from Millipore, Sigma (Massachusetts, USA).

Cardiomyoblasts (H9c2) were obtained from ATCC (Virginia, USA). This cell line is derived from embryonic BD1X rat heart tissue that exhibits many of the properties of skeletal muscle. Cells were maintained in Dulbecco's Modified Eagle's Medium (DMEM) from Thermofisher (Massachusetts, USA) supplemented with 10% Fetal Bovine Serum (FBS) and 1% Penicillin-Streptomycin (PS) from Millipore, Sigma (Massachusetts, USA).

Human skeletal muscle cells were obtained from ATCC (Virginia, USA). This cell line is isolated from normal, human skeletal muscle. Cells were maintained in Mesenchymal Stem Cell Basal Medium supplemented with Primary Skeletal Muscle Growth Kit from ATCC (Virginia, USA). This cell line was differentiated in single-component Skeletal Muscle Differentiation Tool from ATCC (Virginia, USA) for 15 days.

**Table1. Ingredients' sources:**

| Name | Company Name and Location |
|---|---|
| Magnesium citrate (Mg citrate) | NOW, Illinois, USA |
| Alpha-Ketoglutaric acid (aKA) | Double Wood Supplements, Pennsylvania, USA |
| Niacinamide | Nutricost, Utah, USA |
| L-glutamine | Bulk Supplements, Nevada, USA |
| L-citrulline | Bulk Supplements, Nevada, USA |
| Sodium pyruvate | Research Products International, Illinois, USA |
| Succinic acid | Chem Center, California, USA |
| L-cysteine | MilliporeSigma, Massachusetts, USA |
| Taurine | Bulk Supplements, Nevada, USA |
| B-complex | Dr. Rath Vitamin B complex Heerlen, The Netherlands |
| L-methionine | MilliporeSigma, Massachusetts, USA |
| Pantothenic acid (vitamin B5) | PureBulk, Inc. Roseburg, OR 97471 |
| Coenzyme Q10 (CoQ10) | Dr Rath International, CA USA |
| L-Ascorbic acid (VC) | Sigma, St. Louis, MO |

Table 2. Mixes (compositions tested in the study). Equal amount of each individual ingredient was combined in each set. The sets are composed of different numbers of ingredients, such as M11 contains 11 ingredients, G13 contains the same ingredients present in M11 plus VC and CoQ10, G12 set contains the same ingredients present in G13 except for B-complex, and Core contains five ingredients as specified in Table 2.

**Table 2. Lists of the compositions of different sets of natural compounds tested in the study.**

| Mix 11 | Mix G12 | Mix G13 | Core Mix |
|---|---|---|---|
| Magnesium citrate | Magnesium citrate | Magnesium citrate | Magnesium citrate |
| Alpha-Ketoglutaric acid | Alpha-Ketoglutaric acid | Alpha-Ketoglutaric acid | Alpha-Ketoglutaric acid |
| Niacinamide | Niacinamide | Niacinamide | |
| L-glutamine | L-glutamine | L-glutamine | |
| L-citrulline | L-citrulline | L-citrulline | L-citrulline |
| Sodium pyruvate | Sodium pyruvate | Sodium pyruvate | |
| Succinic acid | Succinic acid | Succinic acid | |
| B-complex | | B-complex | |
| Taurine | Taurine | Taurine | Taurine |
| L-cysteine | L-cysteine | L-cysteine | |
| L-methionine | L-methionine | L-methionine | |
| | L-Ascorbic acid | L-Ascorbic acid | |
| | Coenzyme Q10 | Coenzyme Q10 | |

All ingredients were dissolved in dimethyl sulfoxide (DMSO) from MilliporeSigma (MA, USA) to a final concentration of 20 mg /ml, and then all ingredients separately diluted with ultra purified water without (control) or to a 1.0 mg/ml working solutions before applying to cells. Working solutions were diluted in DMEM to final concentrations of 1.0 µg/ml. Mitobiogenesis assay. MitoBiogenesis^{™} In-Cell ELISA Kit (Colorimetric) was purchased from Abcam (Cambridge, UK) and used to assessed mitobiogenesis in the test cell lines. Briefly, cells were grown to confluence in 96-well plates. The day of the assay cells were treated with the test ingredients or their mixtures at concentrations indicated on individual graphs and incubated for 24 hours at 37°C. Media was removed and cells were washed with 1 x Phosphate Buffered Saline (1 x PBS) and then fixed with 4% paraformaldehyde and processed as per the protocol provided with the kit. This assay was used to quantify two mitochondrial proteins: subunit I of Complex IV (COX-I) and the 70 kDa subunit of Complex II (SDH-A). Color intensity was measured at 405 nm for SDH-A and after emptying the wells, the HRP substrate was added, and color development was measured at 600 nm for COX-I.

ATP production assay. Cells were plated on 96-well plate in Minimum Essential Medium Eagle (MEM) medium (stripped from any vitamins) supplemented with 10% FBS at 1.0x105 density for 8h prior the experiment to allow their attachment. Next, the culture medium was replaced with MEM without 10% FBS and cells were treated with the test ingredients or their mixtures at concentrations indicated on individual graphs and incubated for 24h at 37°C. Then, the conditioning medium from all wells were discarded and cells were subjected to ATP bioluminescence assays to quantify ATP amount (Sigma, Massachusetts, USA) according to provided protocol with ATP detection mix diluted at 1:4 and added into all wells to perform the measurements immediately to assessed relative chemiluminescence units (RLU) level with multimode plate reader (Tecan Group Ltd., Switzerland).

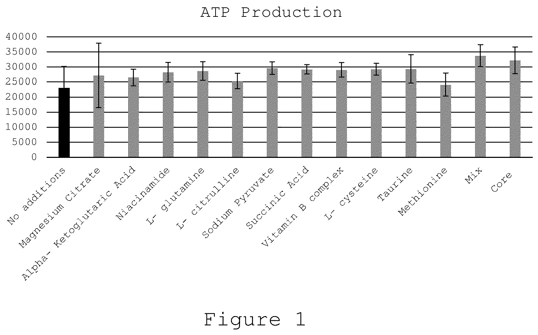

Cellular reactive species (ROS) assay. DCFDA/H2DCFDA Cellular ROS Assay Kit was used for measurement of reactive oxygen species (ROS), purchased from Abcam (Cambridge, UK). Briefly, cells were plated in 96-well plate and grown to confluency. Next, culture medium was exchanged into Minimum Essential Medium Eagle (MEM) medium (stripped from any vitamins) and cells were treated the test ingredients or their mixtures at concentrations indicated on individual graphs and incubated for additional 24h at 37°C. 4h prior the experiment cellular ROS were induced with TBHP compound. One hour prior to completion of the treatment, cells were stained with DCFDA in the same media used for treatment (containing experimental compounds/mixes) in 37°C. Then, plates were transferred to microplate reader without washing to read end point in the presence of compounds and DCFDA with Ex/Em = 485/535 nm. Viability. Survival of test cell lines was quantified by CellTiter-Glo^{®} 2.0 Assay by Promega (Wisconsin, USA). Cells were grown to confluency in 96-well plates and co-treated with the test ingredients or their mixtures at concentrations indicated on individual graphs and 1.3 mM H2O2, and incubated for 24 hours at 37°C. The cells were then processed as per the protocol provided with the CellTiter-Glo Luminescence Cell Viability Assay kit (Promega, Madison, WI). Control cells were not treated with any compound; negative control was treated with 1.3 mM H2O2 only. Figure 1 shows Human myocyte cells were grown to confluency and treated with test ingredients for 24 hours. The cells were then fixed and processed for ATP determination using CellTiter-Glo^{®} 2.0 as described. The subset of four Mix components (magnesium citrate, alpha keto glutaric acid, citrulline and taurine) was designated as a Core.

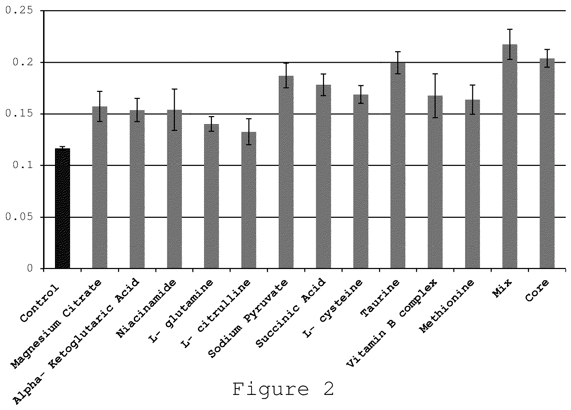

Figure 2 shows the results of an experiment using rat cardiac myoblasts cells grown to confluency and treated with test ingredients for 24 hours. The cells were then fixed and processed as described in Materials and Methods. This assay was used to quantify mitochondrial DNA coded protein: subunit I of Complex IV (COX-I) and subunit I of succinate dehydrogenase complex (SDH-A) nuclear encoded protein using MitoBiogenesis^{™} In-Cell ELISA Kit. The subset of four Mix components (magnesium citrate, alpha keto glutaric acid, citrulline and taurine) was designated as a Core.

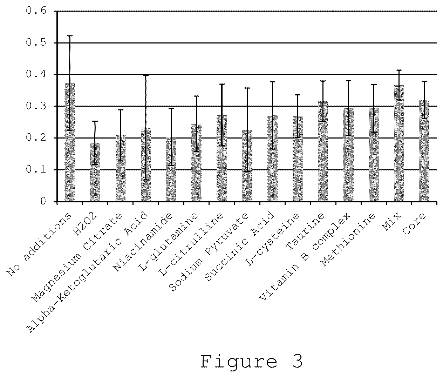

Figure 3 shows the results of microglial cells exposed to test compounds for 24 hours following by Hydrogen Peroxide for 1 hour. After removing H2O2 the cells were incubated in DMEM supplemented with 1% BSA for a further 24 hours and their viability was evaluated at 570nm as described in Materials and Methods. The subset of four Mix components (magnesium citrate, alpha keto glutaric acid, citrulline and taurine) was designated as a Core (mix).

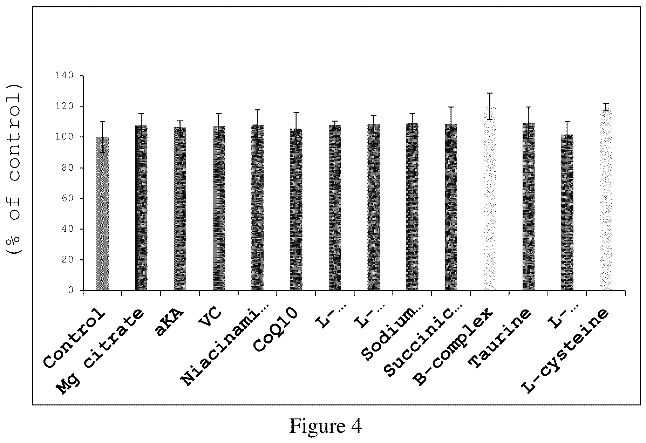

Figure 4 shows ATP synthesis in human cardiac cells are shown with individual ingredients. Cells were grown to confluency and treated with test ingredients (2 µg/ml each) for 24 hours.

Changes expressed in % compared to control. Columns 11 showing B-Complex and L- Cysteine in column 14 indicate statistical significance: p<0.05-0.1.

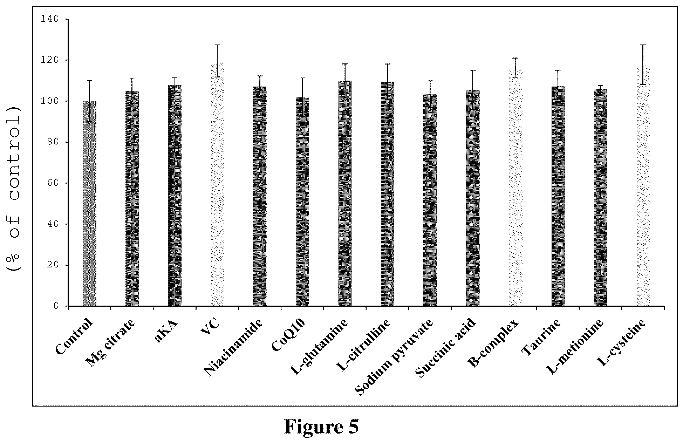

Figure 5 shows ATP synthesis in human skeletal muscle cells (differentiated) as and shows indicate statistical significance: p<0.05-0.1 for Vitamin C (VC), B-Complex and L-Cysteine. Cells were grown to confluency and treated with test ingredients (2 µg/ml each) for 24 hours.

Changes expressed in % compared to control.

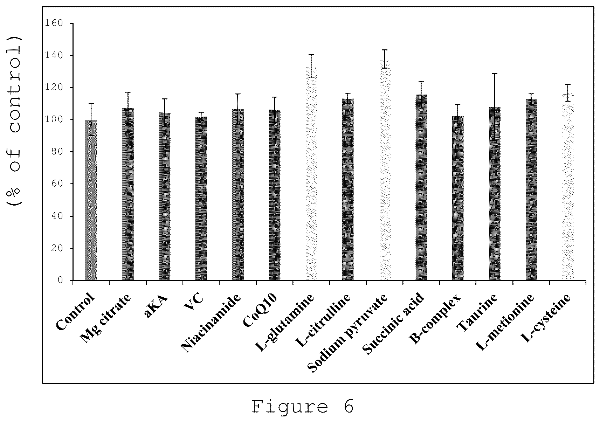

Figure 6 shows ATP synthesis in mouse microglial cells indicate statistical significance: p<0.05-0.1 for L-Glutamate, sodium pyruvate and L-Cysteine. Cells were grown to confluency and treated with test ingredients (2 µg/ml each) for 24 hours. Changes expressed in % compared to control.

The subsequent figures show Effects of individual components present in all these compositions on mitobiogenesis in different types of human cells:
- human cardiomyocytes (AC16)
- human skeletal muscle cells (differentiated)
- mouse immortalized microglial cells
- human liver cells (HepG2)
- rat cardiomyoblasts (H9c2)

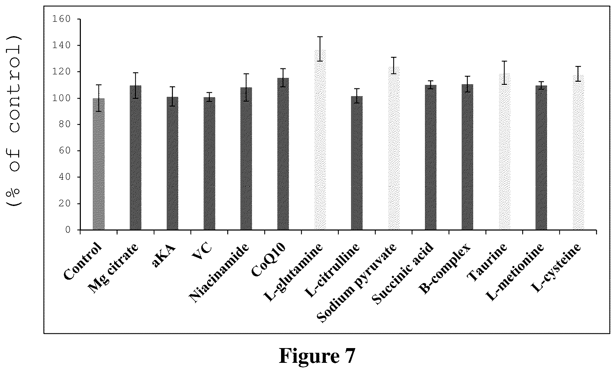

Mitobiogenesis in various types of cells was evaluated by measuring mitochondrial DNA encoded protein COX-I and nuclear DNA encoded protein SDH-A which is relocated to mitochondria and present in the inner mitochondrial membrane as Complex II respiratory chain. Ratio of Cox-I to SDH-A is being used as the representation of mitochondrial biogenesis. Figure 7 shows ATP synthesis in human liver cells (HepG2) as indicate statistical significance: p<0.05-0.1 for L-glutamate, sodium pyruvate, Taurine, and L-Cysteine. Cells were grown to confluency and treated with test ingredients (2 µg/ml each) for 24 hours. Changes expressed in % compared to control.

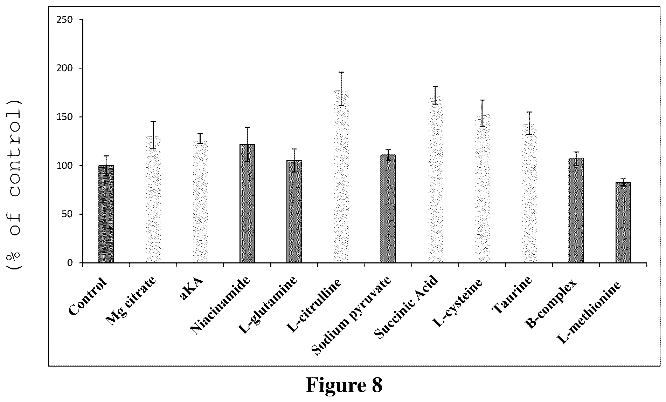

Figure 8 shows ATP synthesis in rat cardio-myoblasts (H9c2) as indicate statistical significance: p<0.05-0.1 for Magnesium citrate, Alpha-Ketoglutaric Acid, L-citruline, succinic acid, Taurine and L-cysteine. Cells were grown to confluency and treated with test ingredients (2 µg/ml each) for 24 hours. Changes expressed in % compared to control.

Effects of individual components present in all these compositions on mitobiogenesis in different types of human cells:
- human cardiomyocytes (AC16)
- human skeletal muscle cells (differentiated)
- mouse immortalized microglial cells
- human liver cells (HepG2)
- rat cardiomyoblasts (H9c2)

Mitobiogenesis in various types of cells was evaluated by measuring mitochondrial DNA encoded protein COX-I and nuclear DNA encoded protein SDH-A which is relocated to mitochondria and present in the inner mitochondrial membrane as Complex II respiratory chain. Ratio of Cox-I to SDH-A is being used as the representation of mitochondrial biogenesis. Various individual ingredients make a Mix. Mixes are named as Mix 11, G12, G13 and core and compared for different functions. Doses of individual ingredients in the various mixes were calculated for human and mammal consumption as follows.

**Table 3: Mix composition calculation.**

| **Ingredients in Bioenergy** | **Doses** |
|---|---|
| Magnesium Citrate | 10-30,000 mg |
| Alpha-Ketoglutaric Acid | 10- 30 ,000 mg |
| Niacinamide | 1 - 10,000 mg |
| L Glutamine | 10 - 50,000 mg |
| L Citrulline | 10 - 20,000mg |
| Sodium Pyruvate | 10-50,000mg |
| Succinic acid | 10 - 30,000mg |
| Vitamin B complex (DR RATH B-Complex) | B1-2.4 mg-48 mg |
| | B2-3.7 mg- 74mg |
| | B3- 33 mg -670 mg |
| | B5-15 mg-300 mg |
| | B6-3.2 mg-64 mg |
| | B12-6 mcg- 120 mcg |
| | Folic acid -50 mcg-10,000 mcg |
| | Biotin 110 mcg-2200 mcg |
| Taurine | 10-10,000mg |
| Cysteine | 10-20,000mg |
| Methionine | 10-20,000mg |
| L-Ascorbic Acid | 5 - 50,000mg |
| Coenzyme Q10 | 1-3,000mg |

Several formulations are prepared to make said micronutrient composition to be administered to a mammal. Physiological doses are calculated and each dose is administered to enhance, cure or manage efficient functioning of each step of the metabolic pathway for disease prevention, faster recovery from injury and overall wellness. Several ingredients as shown in Table 2 and optionally adding one or combination of pharmaceutically acceptable carriers or excipient or liquefied propellant or buffer or pH regulator or stabilizer or coating or flavoring agent is formulated as a tablet, coated tablet, capsule, pill, intranasal, lozenges, emulsion, pastilles, suppository, paste or injectable solution were used for treating a mammal.

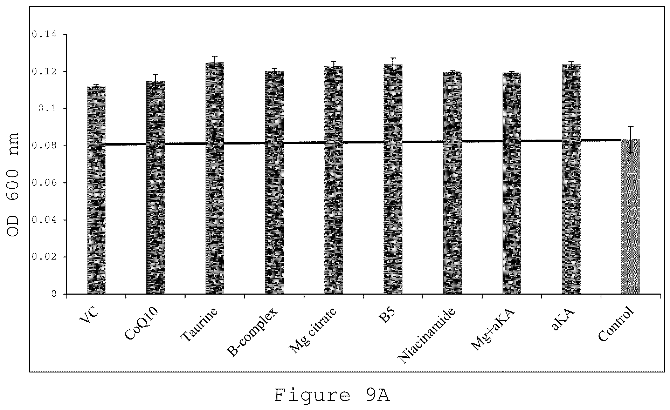

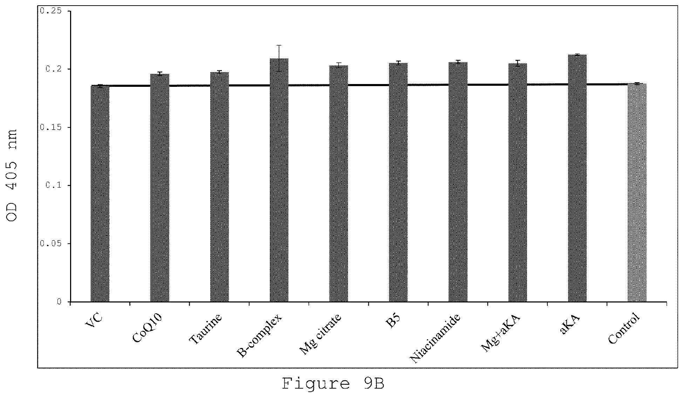

Figure 9A and Figure 9B shows effects of individual compounds on mitochondria formation in human cardiac myocytes (AC16) evaluated by mitochondria encoded COX-I protein and nuclear encoded protein SDH-A. Cells were grown to confluency and exposed to test ingredients at concentrations 2 µg/ml for 24 hours as described in Materials and Methods. Compared to control all ingredients have beneficial effect on COX-1 and SDH-A.

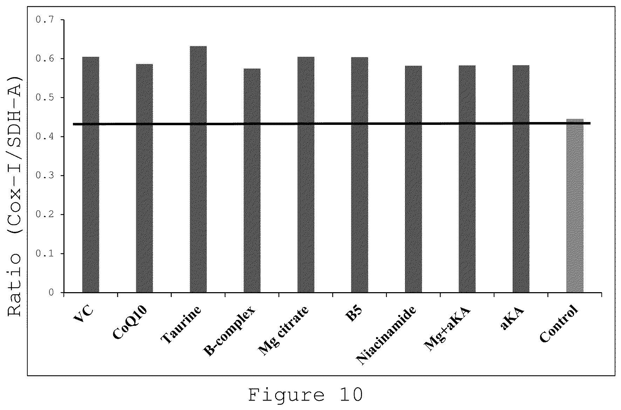

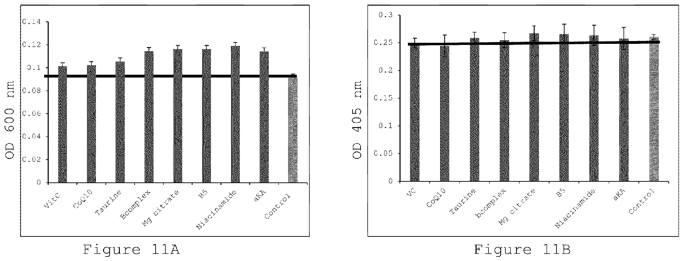

Figure 10 shows Cox1/SDH-A Ratios: human cardiomyocytes (AC16) when exposed to individual nutrients. Figure 11A and Figure 11B shows the effects of individual compounds on mitochondria formation in differentiated human skeletal muscle cells evaluated by mitochondria encoded COX-I protein and nuclear encoded protein SDH-A. Cells were grown to confluency and exposed to test ingredients at 2 µg/ml for 24 hours as described.

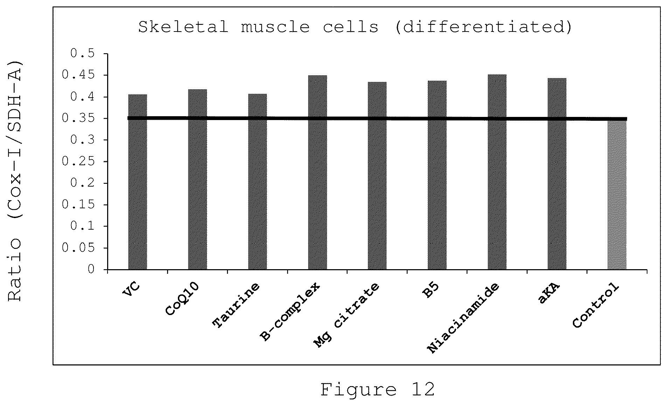

Figure 12 shows a study of Cox1/SDH-A Ratios: human differentiated skeletal muscle cells.

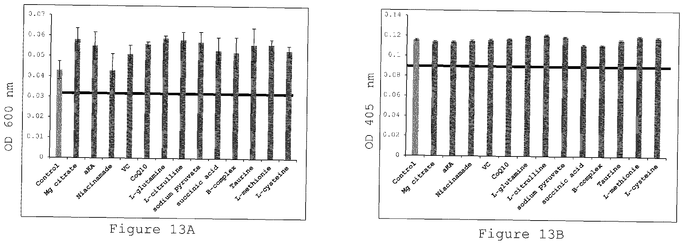

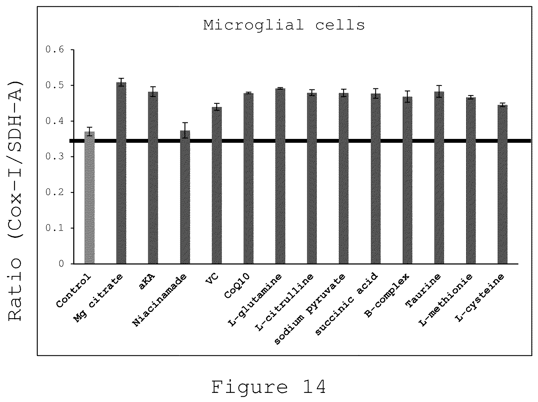

Figure 13A and Figure 13B shows evaluation of individual ingredients effects on mitochondria formation in mouse immortalized microglial cells. The effects of individual compounds on mitochondria formation in microglial cells by evaluating mitochondrial encoded protein COX-I (Fig. 13A) and nuclear encoded SDH-A (Fig. 13B). Cells were grown to confluency and exposed to test ingredients at 2 µg/ml for 24 hours as described. Figure 14 shows Changes in ratios of Cox-I to SDH-A in mouse immortalized microglial cells exposed to individual nutrients. Most individual ingredients have a higher value than control.

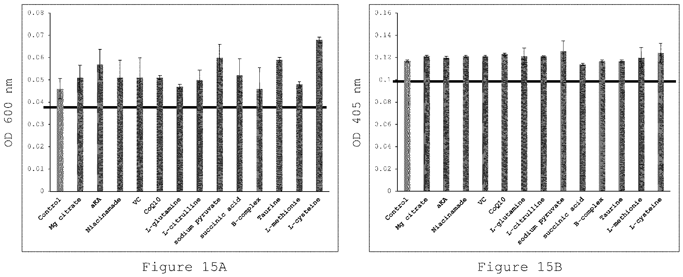

Figure 15A and Figure 15B shows the effects of individual compounds on mitochondria formation in human liver cells (HepG2) evaluated by mitochondria encoded COX-I protein and nuclear encoded protein SDH-A. Cells were grown to confluency and exposed to test ingredients at 2 µg/ml for 24 hours. COX-1 has more reaction than SDH-A to the individual ingredients.

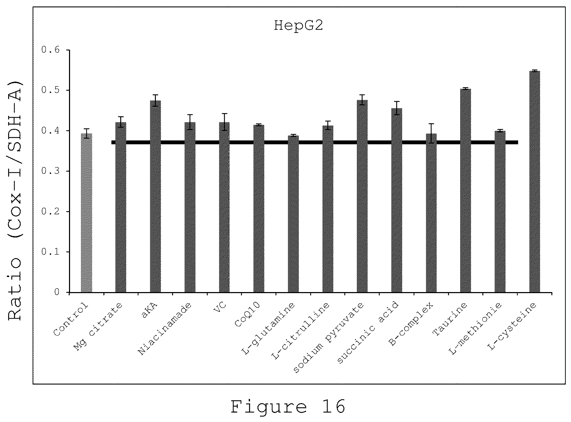

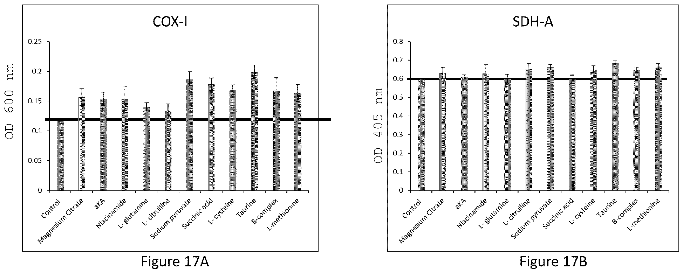

Figure 16 shows changes in ratios of Cox-I to SDH-A in human liver cells HepG2 exposed to individual nutrients. L-Cysteine seems to have significant effect. Figure 17A and Figure 17B shows the evaluation of individual ingredients effects on mitochondria formation in rat cardiomyoblast cells (H9c2) results. Effects of test ingredients on mitochondrial DNA coded protein COX-I and nuclear encoded SDH-A in rat cardiac myoblasts (H9c2) grown to confluency and treated with test ingredients at 2 µg/ml for 24 hours as specified in materials and methods.

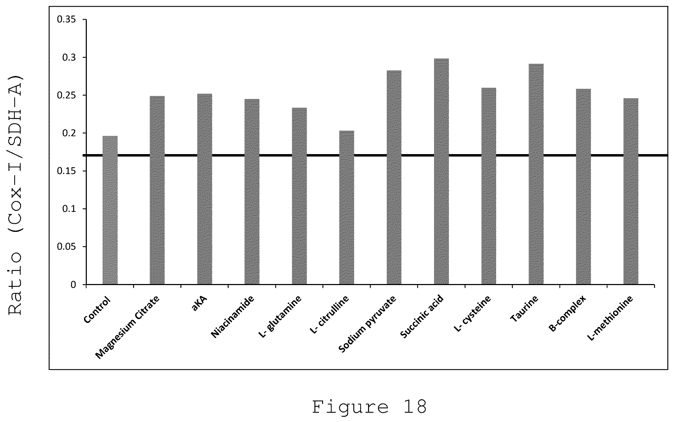

Figure 18 shows changes in ratios of Cox-I to SDH-A in rat cardiomyoblasts (H9c2) exposed to individual nutrients. The COX/SDH ratio is important because it measures mitochondrial respiratory function and helps diagnose mitochondrial diseases by identifying cells with dysfunction. By comparing the activity of COX (cytochrome c oxidase), which is encoded by both nuclear and mitochondrial DNA (mtDNA), to SDH (succinate dehydrogenase), which is encoded only by nuclear DNA, the ratio reveals issues specifically in the mtDNA-encoded parts of the respiratory chain. A low ratio can indicate a problem with the mtDNA, as seen in mitochondrial myopathies.

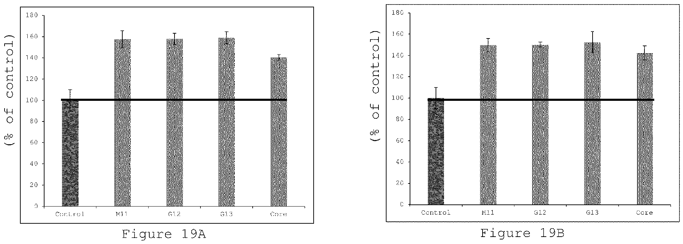

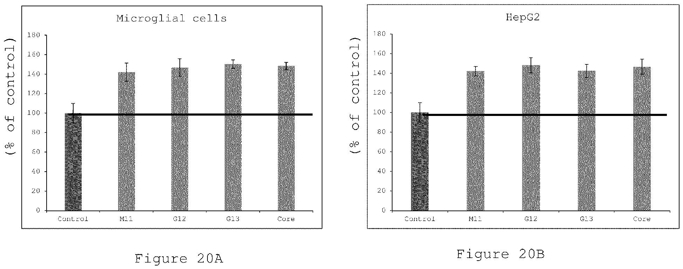

The subsequent figures show mixes being used on cell types described above and their effect on ATP synthesis and effect on mitobiogenesis. Figure 19A and Figure 19B shows effects of nutrient compositions M11, G12, G13 and Core on ATP synthesis in human cardiomyocytes and differentiated human skeletal muscle cells. Cells were grown to confluency and exposed to test combinations at concentrations indicated in Materials and Methods. Changes are expressed in % compared to control. The results are expressed as percentage of control. Figure 20A and Figure 20B shows effects of nutrient compositions M11, G12, G13 and Core on ATP synthesis in mouse immortalized microglial cells and human liver HepG2 cells. Cells were grown to confluency and exposed to test combinations at concentrations indicated in materials and methods. Changes are expressed in % compared to control.

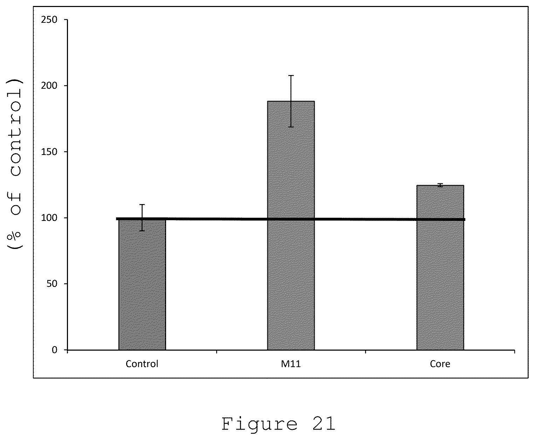

Figure 21 shows effects of nutrient compositions M11 and Core on ATP synthesis in rat cardiomyoblasts (H9c2). Cells were grown to confluency and exposed to test combinations at 22 µg/ml (M11) and 8 µg/ml (Core) concentrations as described in Materials and Methods. Changes are expressed in % compared to control.

**Table 4: Increase in ATP production (%) by test mixes in different cell types compared to control:**

| **Cell type** | **M11** | **G12** | **G13** | **Core** |
|---|---|---|---|---|
| Skeletal muscle | 50% | 50% | 52% | 42% |
| Human cardiomyocytes | 58% | 58% | 59% | 41% |
| Microglial cells | 42% | 47% | 50% | 48% |
| Liver cells | 42% | 48% | 42% | 47% |
| Rat cardiomyoblasts | 88% | - | - | 25% |

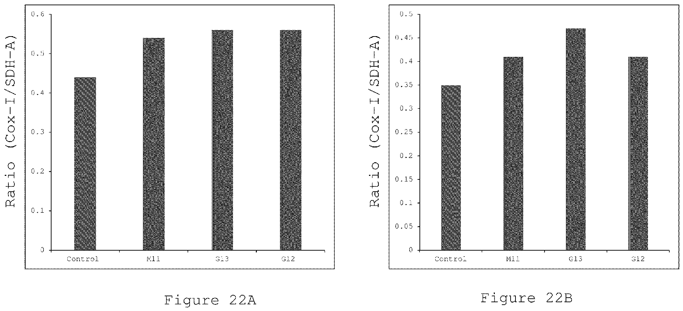

Figure 22A and Figure 22B shows the effects of M11, G12 and G13 compared to control on mitobiogenesis expressed as ratio of COX-I (mitochondria encoded gene) to SDH-A (nucleus encoded) in human cardiomyocytes and differentiated human skeletal muscle cells as described in Material and Methods.

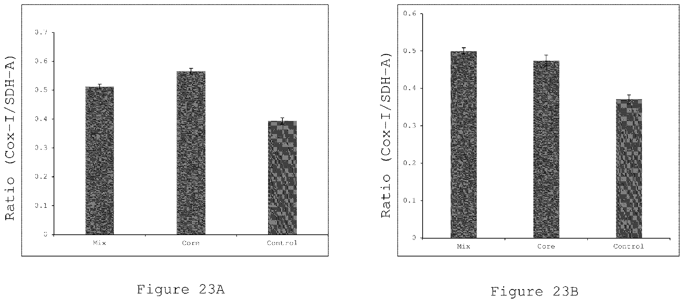

Figure 23A and Figure 23B shows the effects of Mix (G13) and Core compared to control on mitobiogenesis expressed as ratio of COX-I (mitochondria encoded gene) to SDH-A (nucleus encoded) in human liver cells (HepG2) and mouse microglial cells as described in material and methods.

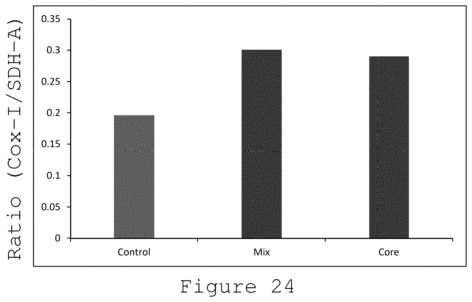

Figure 24 shows the effects of Mix 11 and Core compared to control on mitobiogenesis expressed as ratio of COX-I (mitochondria encoded gene) to SDH-A (nucleus encoded) in rat cardiomyoblasts (H9c2) as described in Material and Methods.

The following figures show other metabolic effects of nutrients applied individually and in combinations: for example-
- Quenching reactive oxygen species (ROS) by individual components and test Mixes.
- Effects of individual compounds, Mix 11 and Core on viability of microglial cells exposed to hydrogen peroxide.

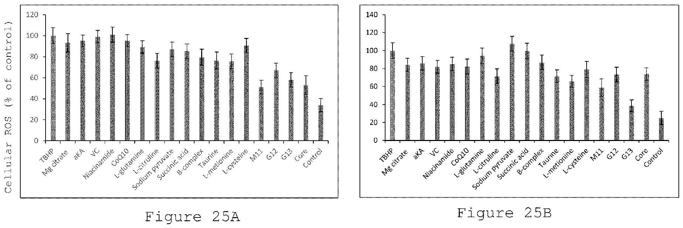

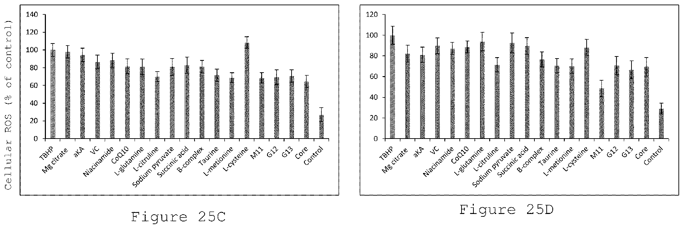

Figure 25A, Figure 25B, Figure 25C and Figure 25D shows effects of individual compounds and their combinations on quenching of cellular reactive species (ROS) in four different cell lines. ROS were induced with 50 µM TBHP compound. Cells were grown to confluency and exposed to test compounds at 2.0 µg/ml or their combinations at 22 µg/ml (M11), 24 µg/ml (G12), 26 µg/ml (G13) and 8 µg/ml (Core) concentrations as described in Materials and Methods.

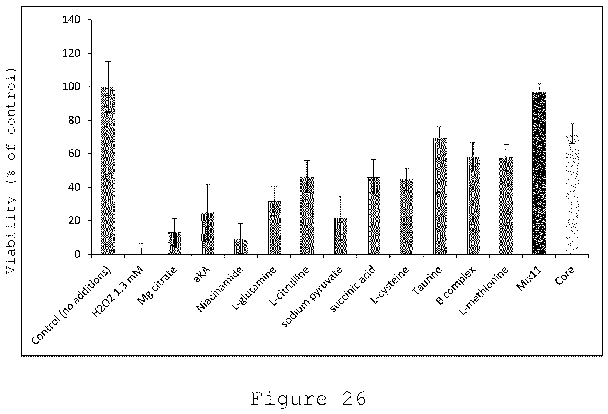

Figure 26 shows the effects of individual ingredients and their defined combinations in Mix11 and Core on viability of mouse immortalized microglial cells exposed to the test compounds (0.75 µg/ml each), Mix11 (22 µg/ml), and Core (8 µg/ml) for 24 hours followed by hydrogen peroxide treatment as described in Material and Methods.

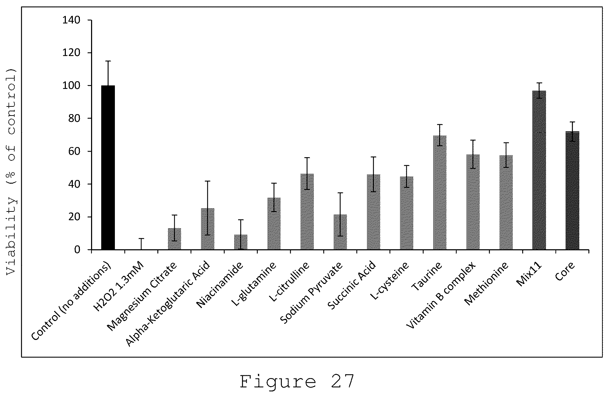

Figure 27 shows the effects of individual natural compounds and their defined combinations in Mix 11 and Core on viability of mouse immortalized microglial cells exposed to the test compounds (0.75 µg/ml) for 24 hours followed by hydrogen peroxide treatment.

The Mixes comprise of one of a Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine, L- Cysteine or combination thereof. In one embodiment, a deletion or addition of other ingredients are combined. Formulations of these mixes are produced suitable for oral administration may be in the form of capsules, cachets, pills, tablets, lozenges (using flavored bases, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin or sucrose and acacia), each containing a predetermined amount of a subject composition as an active ingredient. Subject compositions may also be administered as a bolus, electuary or paste.

When an oral solid drug product is prepared, micronutrient composition is mixed with an excipient (and, if necessary, one or more additives such as a binder, a disintegrant, a lubricant, a coloring agent, a sweetening agent, and a flavoring agent), and the resultant mixture is processed through a routine method, to thereby produce an oral solid drug product such as tablets, coated tablets, granules, powder or capsules. Additives may be those generally employed in the art. Examples of excipients include lactate, sucrose, sodium chloride, glucose, starch, calcium carbonate, kaolin, microcrystalline cellulose and silicic acid. Binders include water, ethanol, propanol, simple syrup, glucose solution, starch solution, liquefied gelatin, carboxymethylcellulose, hydroxypropyl cellulose, hydroxypropyl starch, methyl cellulose, ethyl cellulose, shellac, calcium phosphate and polyvinyl pyrrolidone. Disintegrants include dried starch, sodium arginate, powdered agar, sodium hydroxy carbonate, calcium carbonate, sodium lauryl sulfate, monoglyceryl stearate and lactose. Lubricants include purified talc, stearic acid salts, borax and polyethylene glycol. Sweetening agents include sucrose, orange peel, citric acid and tartaric acid.

When a liquid drug product for oral administration is prepared, micronutrient composition is mixed with an additive such as a sweetening agent, a buffer, a stabilizer, or a flavoring agent, and the resultant mixture is processed through a routine method, to produce an orally administered liquid drug product such as an internal solution medicine, syrup or elixir. Examples of the sweetening agent include vanillin; examples of the buffer include sodium citrate; and examples of the stabilizer include tragacanth, acacia, and gelatin.

For the purposes of transdermal (e.g., topical) administration, dilute sterile, aqueous or partially aqueous solutions (usually in about 0.1% to 5% concentration), otherwise similar to the above parenteral solutions, may be prepared with micronutrient composition.

Formulations containing micronutrient composition for rectal or vaginal administration may be presented as a suppository, which may be prepared by mixing a subject composition with one or more suitable non-irritating carriers, comprising, for example, cocoa butter, polyethylene glycol, a suppository wax or a salicylate, which is solid at room temperature, but liquid at body temperature and, therefore, will melt in the appropriate body cavity and release the encapsulated compound(s) and composition(s). Formulations that are suitable for vaginal administration also include pessaries, tampons, creams, gels, pastes, foams or spray formulations containing such carriers as are known in the art to be appropriate.

A targeted-release portion for capsules containing micronutrient composition can be added to the extended-release system by means of either applying an immediate-release layer on top of the extended release core; using coating or compression processes, or in a multiple-unit system such as a capsule containing extended- and immediate- release beads.

When used with respect to a micronutrient composition, the term "sustained release" is art recognized. For example, a therapeutic composition that releases a substance over time may exhibit sustained-release characteristics, in contrast to a bolus type administration in which the entire amount of the substance is made biologically available at one time. In particular embodiments, upon contact with body fluids, including blood, spinal fluid, mucus secretions, lymph or the like, one or more of the pharmaceutically acceptable excipients may undergo gradual or delayed degradation (e.g., through hydrolysis), with concomitant release of any material incorporated therein, e.g., a therapeutic and/or biologically active salt and/or composition, for a sustained or extended period (as compared with the release from a bolus). This release may result in prolonged delivery of therapeutically effective amounts of any of the therapeutic agents disclosed herein.

Current efforts in the area of drug delivery include the development of targeted delivery, in which the drug is only active in the target area of the body (for example, mucous membranes such as in the nasal cavity), and sustained-release formulations, in which the micronutrient composition is released over a period of time in a controlled manner from a formulation. Types of sustained release formulations include liposomes, drug-loaded biodegradable microspheres and micronutrient composition polymer conjugates.

Delayed-release dosage formulations are created by coating a solid dosage form with a film of a polymer, which is insoluble in the acid environment of the stomach, but soluble in the neutral environment of the small intestine. The delayed-release dosage units can be prepared, for example, by coating a micronutrient composition with a selected coating material. The micronutrient composition may be a tablet for incorporation into a capsule, a tablet for use as an inner core in a "coated core" dosage form, or a plurality of drug-containing beads, particles or granules, for incorporation into either a tablet or a capsule. Preferred coating materials include biodegradable, gradually hydrolysable, gradually water-soluble, and/or enzymatically degradable polymers, and may be conventional "enteric" polymers. Enteric polymers, as will be appreciated by those skilled in the art, become soluble in the higher pH environment of the lower gastrointestinal tract, or slowly erode as the dosage form passes through the gastrointestinal tract, while enzymatically degradable polymers are degraded by bacterial enzymes present in the lower gastrointestinal tract, particularly in the colon. Alternatively, a delayed-release tablet may be formulated by dispersing a drug within a matrix of a suitable material such as a hydrophilic polymer or a fatty compound. Suitable hydrophilic polymers include, but are not limited to, polymers or copolymers of cellulose, cellulose ester, acrylic acid, methacrylic acid, methyl acrylate, ethyl acrylate and vinyl or enzymatically degradable polymers or copolymers as described above. These hydrophilic polymers are particularly useful for providing a delayed-release matrix. Fatty compounds for use as matrix material include, but are not limited to, waxes (e.g., carnauba wax) and glycerol tri-stearate. Once the active ingredient is mixed with the matrix material, the mixture can be compressed into tablets.

A pulsed-release dosage is one that mimics a multiple dosing profile without repeated dosing and typically allows at least a twofold reduction in dosing frequency as compared with the drug presented as a conventional dosage form (e.g., as a solution or prompt drug-releasing, conventional solid dosage form). A pulsed-release profile is characterized by a time period of no release (lag time) or reduced release, followed by rapid drug release. These can be formulated for critically ill patients using instant micronutrient composition.

The phrases "parenteral administration" and "administered parenterally" as used herein refer to modes of administration other than enteral and topical, such as injections, and include without limitation intravenous, intramuscular, intrapleural, intravascular, intrapericardial, intra-arterial, intrathecal, intracapsular, intra-orbital, intracardiac, intradermal, intraperitoneal, transtracheal, subcutaneous, subcuticular, intra-articular, subcapsular, subarachnoid, intraspinal and intra-stemal injection and infusion.

Certain micronutrient composition disclosed herein, suitable for parenteral administration, comprise one or more subject compositions in combination with one or more pharmaceutically acceptable sterile, isotonic, aqueous, or non-aqueous solutions, dispersions, suspensions or emulsions, or sterile powders, which may be reconstituted into sterile injectable solutions or dispersions just prior to use, and which may contain antioxidants, buffers, bacteriostats, solutes that render the formulation isotonic within the blood of the intended recipient, or suspending or thickening agents.

When an injection product is prepared, micronutrient composition is mixed with an additive such as a pH regulator, a buffer, a stabilizer, an isotonicity agent or a local anesthetic, and the resultant mixture is processed through a routine method, to thereby produce an injection for subcutaneous injection, intramuscular injection, or intravenous injection. Examples of the pH regulator or buffer include sodium citrate, sodium acetate and sodium phosphate; examples of the stabilizer include sodium pyrosulfite, EDTA, thioglycolic acid, and thiolactic acid; examples of the local anesthetic include procaine hydrochloride and lidocaine hydrochloride; and examples of the isotonicity agent include sodium chloride and glucose.

Adjuvants are used to enhance the immune response. Various types of adjuvants are available. Haptens and Freund's adjuvant may also be used to produce water-in-oil emulsions of immunogens.

The phrase "pharmaceutically acceptable" is art recognized. In certain embodiments, the term includes compositions, polymers and other materials and/or dosage forms that are within the scope of sound medical judgment, suitable for use in contact with the tissues of mammals, both human beings and animals, without excessive toxicity, irritation, allergic response or other problem or complication, commensurate with a reasonable benefit-risk ratio.

The phrase "pharmaceutically acceptable carrier" is art recognized, and includes, for example, pharmaceutically acceptable materials, compositions or vehicles, such as a liquid or solid filler, diluent, solvent or encapsulating material involved in carrying or transporting any subject composition from one organ or portion of the body, to another organ or portion of the body. Each carrier must be "acceptable" in the sense of being compatible with the other ingredients of a subject composition, and not injurious to the patient. In certain embodiments, a pharmaceutically acceptable carrier is non-pyrogenic. Some examples of materials that may serve as pharmaceutically acceptable carriers include: (1) sugars, such as lactose, glucose and sucrose; (2) starches, such as corn starch and potato starch; (3) cellulose and its derivatives, such as sodium carboxymethyl cellulose, ethyl cellulose and cellulose acetate; (4) powdered tragacanth; (5) malt; (6) gelatin; (7) talc; (8) cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. Throughout the application micronutrient pharmaceutical composition is used as composition, micronutrient composition as an alternate.

In certain embodiments, the micronutrient compositions described herein are formulated in a manner such that said compositions will be delivered to a mammal in a therapeutically effective amount, as part of a prophylactic, preventive or therapeutic treatment to protect, improve and enhance bioenergy in a cell for mammal. In certain embodiments, the dosage of the micronutrient pharmaceutical composition, which may be referred to as therapeutic composition provided herein, may be determined by reference to the plasma concentrations of the therapeutic composition or other encapsulated materials.

The therapeutic micronutrient composition provided by this application may be administered to a subject in need of treatment by a variety of conventional routes of administration, including orally, topically, parenterally, e.g., intravenously, subcutaneously or intramedullary. Further, the therapeutic compositions may be administered intranasally, as a rectal suppository, or using a "flash" formulation, i.e., allowing the medication to dissolve in the mouth without the need to use water. Furthermore, the compositions may be administered to a subject in need of treatment by controlled-release dosage forms, site-specific drug delivery, transdermal drug delivery, patch-mediated drug delivery (active/passive), by stereotactic injection, or in nanoparticles.

Expressed in terms of concentration, an active ingredient can be present in the therapeutic compositions of the present invention for localized use via the cutis, intranasally, pharyngolaryngeally, bronchially, intravaginally, rectally or ocularly. For use as aerosols, the active ingredients can be packaged in a pressurized aerosol container together with a gaseous or liquefied propellant, for example dichlorodifluoromethane, carbon dioxide, nitrogen, propane and the like, with the usual adjuvants such as cosolvents and wetting agents, as may be necessary or desirable. The most common routes of administration also include the preferred transmucosal (nasal, buccal/sublingual, vaginal, ocular and rectal) and inhalation routes.

In addition, in certain embodiments, the subject micronutrient composition of the present application may be lyophilized or subjected to another appropriate drying technique such as spray drying. The subject compositions may be administered once or may be divided into a number of smaller doses to be administered at varying intervals of time, depending in part on the release rate of the compositions and the desired dosage.

Formulations useful in the methods provided herein include those suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of a subject micronutrient composition that may be combined with a carrier material to produce a single dose may vary depending upon the subject being treated and the particular mode of administration. Physiological dose levels for mammalian consumption were calculated based on various factors which include type of administration, species dependency and mode of action, such as transdermal vs oral. The range disclosed includes those factors along with scientific calculations. The range may differ within the range as well depending on formulations and species. Drug formulations suitable for these administration routes can be produced by adding one or more pharmacologically acceptable carrier to the agent and then treating the micronutrient composition through a routine process known to those skilled in the art. The mode of administration includes, but is not limited to, non-invasive peroral, topical (for example, transdermal), enteral, transmucosal, targeted delivery, sustained-release delivery, delayed release, pulsed release and parenteral methods. Peroral administration may be administered both in liquid and dry state.

The therapeutically acceptable amount described herein may be administered in inhalant or aerosol formulations. The inhalant or aerosol formulations may comprise one or more agents, such as adjuvants, diagnostic agents, imaging agents, or therapeutic agents useful in inhalation therapy. The final aerosol formulation may, for example, contain 0.005-90% w/w, for instance 0.005-50%, 0.005-5% w/w, or 0.01-1.0% w/w, of medicament relative to the total weight of the formulation.

Examples of suitable aqueous and non-aqueous carriers that may be employed in the micronutrient composition include water, ethanol, polyols (such as glycerol, propylene glycol, polyethylene glycol and the like), and suitable mixtures thereof, vegetable oils such as olive oil, and injectable organic esters such as ethyl oleate. Proper fluidity may be maintained, for example by the use of coating materials such as lecithin, by the maintenance of the required particle size in the case of dispersions, and using surfactants.

### INDUSTRIAL USE

Thus, in this study we prove that micronutrient composition plays a decisive role in regulating the joint mechanism by enhancing ECM related components in individuals. With optimum combination of natural ingredients and micronutrients formulated in various forms for a suitable consumption to prevent and treat arthritic patient is described.

Summarizing the findings of the present invention, the following embodiments are particularly preferred:
Embodiment 1 relates to a pharmaceutical composition comprising at least one ingredient selected from the group consisting of Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine, L- Cysteine and combination thereof, preferably wherein the pharmaceutical composition comprises Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine and L- Cysteine.

Embodiment 2 relates to a pharmaceutical composition as described in embodiment 1, wherein the pharmaceutical composition comprises or consists of a Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000 mg, Coenzyme Q10 in the range of 1-3,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg, Folic acid in the range of 50 mcg-10,000 mcg, Biotin in the range of 110 mcg-2200 mcg, and Taurine in the range of 10-10,000 mg, L-Methionine in the range of 10-20,000 mg, L- Cysteine in the range of 10-20,000 mg.

Embodiment 3 relates to a pharmaceutical composition comprising at least one ingredient selected from the group consisting of Magnesium Citrate, Alpha-Ketoglutaric Acid, Niacinamide, L Glutamine, L Citrulline, Sodium Pyruvate, Succinic acid, Vitamin B Complex, Taurine, L-Methionine, L- Cysteine and a combination thereof, preferably comprising Magnesium Citrate, Alpha-Ketoglutaric Acid, Niacinamide, L Glutamine, L Citrulline, Sodium Pyruvate, Succinic acid, Vitamin B Complex, Taurine, L-Methionine, and L- Cysteine, more preferably consisting of Magnesium Citrate, Alpha-Ketoglutaric Acid, Niacinamide, L Glutamine, L Citrulline, Sodium Pyruvate, Succinic acid, Vitamin B Complex, Taurine, L-Methionine, and L- Cysteine.

Embodiment 4 relates to a pharmaceutical composition comprising at least one ingredient selected from the group consisting of Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, L- Cysteine and combination thereof, preferably comprising Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, and L-Cysteine, more preferably consisting of Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, and L- Cysteine.

Embodiment 5 relates to a pharmaceutical composition comprising at least one ingredient selected from the group consisting of Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine, L- Cysteine and combination thereof, preferably comprising Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine and L- Cysteine, more preferably consisting of Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine and L-Cysteine.

Embodiment 6 relates to a pharmaceutical composition comprising at least one ingredient selected from the group consisting of Magnesium Citrate, Alpha-Ketoglutaric Acid, L Citrulline and Taurine, preferably comprising Magnesium Citrate, Alpha-Ketoglutaric Acid, L Citrulline and Taurine.

Embodiment 7 relates to the pharmaceutical composition as described in any one of embodiments 1 to 6 additionally comprising one or a combination of pharmaceutically acceptable carriers or excipient or liquefied propellant or buffer or pH regulator or stabilizer or coating or flavoring agent, wherein the pharmaceutical composition is formulated as a tablet, coated tablet, capsule, pill, intranasal, lozenges, emulsion, pastilles, suppository, powder, paste or injectable solution.

Embodiment 8 relates to the pharmaceutical composition of any one of embodiments 1 to 7, wherein the pharmaceutical composition comprises or consists of a Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000 mg, Coenzyme Q10 in the range of 1-3,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg, Folic acid in the range of 50 mcg-10,000 mcg, Biotin in the range of 110 mcg-2200 mcg, and Taurine in the range of 10-10,000 mg, L-Methionine in the range of 10-20,000 mg, L- Cysteine in the range of 10-20,000 mg.

Embodiment 9 relates to the pharmaceutical composition of any one of embodiments 1 to 8, further comprising Vitamin C, Vitamin B complex and Coenzyme Q10.

Embodiment 10 relates to the pharmaceutical composition of embodiment 1, wherein the pharmaceutical composition comprises Magnesium Citrate in the range of 10-30,000 mg, Alpha-Ketoglutaric Acid in the range of 10-30 ,000 mg, Niacinamide in the range of 1-10,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Taurine in the range of 10-10,000 mg, L-Methionine in the range of 10-20,000 mg, L- Cysteine in the range of 10-20,000 mg.

Embodiment 11 relates to the pharmaceutical composition of embodiment 1, wherein the pharmaceutical composition comprises Vitamin C as L- Ascorbic acid in the range of 5-50,000 mg, Coenzyme Q10 in the range of 1-3,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, and Biotin in the range of 110 mcg-2200 mcg.

Embodiment 12 relates to the pharmaceutical composition according to any one of embodiments 1 to 11 for use in medicine.

Embodiment 13 relates to the pharmaceutical composition according to any one of embodiments 1 to 11 for use in treating a mammal having a disease which is imposed by an impaired mitobiogenesis ATP production and/or an oxidative stress, preferably wherein the disease is selected from the group consisting of arrhythmia, heart failure, cardiovascular conditions, depression, mental decline and dementia.

Embodiment 14 relates to the pharmaceutical composition for use of embodiment 12 or 13, wherein the pharmaceutical composition is an oral capsule, preferably wherein the oral capsule is to be administered in a dose of one oral capsule once a day, twice a day or three times a day.

Embodiment 15 relates to the pharmaceutical composition for use of any one of embodiments 12 to 14, wherein the pharmaceutical composition comprises or consists of a Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000 mg, Coenzyme Q10 in the range of 1-3,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg, Folic acid in the range of 50 mcg-10,000 mcg, Biotin in the range of 110 mcg-2200 mcg, and Taurine in the range of 10-10,000 mg, L-Methionine in the range of 10-20,000 mg, L- Cysteine in the range of 10-20,000 mg.

Embodiment 16 relates to a pharmaceutical composition according to any one of embodiments 1 to 11 for use in improving bioenergy in a mammal, wherein the pharmaceutical composition preferably comprises at least one ingredient selected from the group consisting of Magnesium Citrate, Alpha-Ketoglutaric Acid, Niacinamide, L Glutamine, L Citrulline, Sodium Pyruvate, Succinic acid, Taurine, L-Methionine, L- Cysteine and combination thereof, and wherein the pharmaceutical composition is more preferably formulated as a tablet, coated tablet, capsule, pill, intranasal, lozenges, emulsion, pastilles, suppository, powder, paste or injectable solution.

Embodiment 17 relates to the pharmaceutical composition for use of embodiment 16, further comprising a mix containing Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, L- Cysteine or combination thereof.

Embodiment 18 relates to the pharmaceutical composition for use of embodiment 16 or 17, further comprising a mix Mix G 12 containing the Magnesium Citrate, Alpha-Ketoglutaric Acid, Niacinamide, L Glutamine, L Citrulline, Sodium Pyruvate, Succinic acid, Vitamin B Complex, Taurine, L-Methionine, and L- Cysteine.

Embodiment 19 relates to the pharmaceutical composition for use of any one of embodiments 16 to 18, further comprising Vitamin C, Vitamin B complex and Coenzyme Q10.

Embodiment 20 relates the pharmaceutical composition for use of any one of embodiments 16 to 19, wherein the pharmaceutical composition comprises Magnesium Citrate in the range of 10-30,000 mg, Alpha-Ketoglutaric Acid in the range of 10-30 ,000 mg, Niacinamide in the range of 1-10,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Taurine in the range of 10-10,000 mg, L-Methionine in the range of 10-20,000 mg, L- Cysteine in the range of 10-20,000 mg.

Embodiment 21 relates the pharmaceutical composition for use of any one of embodiments 16 to 19, wherein the pharmaceutical composition comprises Vitamin C as L- Ascorbic acid in the range of 5-50,000 mg, Coenzyme Q10 in the range of 1-3,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, and Biotin in the range of 110 mcg-2200 mcg.

Embodiment 22 relates the pharmaceutical composition for use according to any one of embodiments 16 to 21, wherein the mammal suffers from a specific disease, wherein the specific disease is due to an impaired mitobiogenesis, ATP production and/or an oxidative stress.

Embodiment 23 relates the pharmaceutical composition for use according to embodiment 22, wherein the disease is selected from the group consisting of arrhythmia, heart failure, cardiovascular conditions, depression, mental decline and dementia

Embodiment 24 relates the A pharmaceutical composition for use treating a specific disease, wherein the specific disease is due to an impaired mitobiogenesis ATP production and/or an oxidative stress, the pharmaceutical composition comprising a Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000 mg, Coenzyme Q10 in the range of 1-3,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg, Folic acid in the range of 50 mcg-10,000 mcg, Biotin in the range of 110 mcg-2200 mcg, and Taurine in the range of 10-10,000 mg, L-Methionine in the range of 10-20,000 mg, L- Cysteine in the range of 10-20,000 mg and is formulated as a tablet, coated tablet, capsule, pill, intranasal, lozenges, emulsion, pastilles, suppository, powder, paste or injectable solution.

Embodiment 25 relates the pharmaceutical composition of any one of embodiments 13 to 24, wherein the wherein the mammal is human.

Embodiment 26 relates the pharmaceutical composition of any one of embodiments 13 to 25, wherein pharmaceutical composition is formulated as oral, nasal, topical, rectal, vaginal, aerosol, parenteral formulation or a combination thereof.

Embodiment 27 relates a method of treating a mammal, comprising; administering a pharmaceutical composition containing one of a Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine, L- Cysteine or combination thereof, for a specific disease and a specific dose; and optionally adding one or combination of pharmaceutically acceptable carriers or excipient or liquefied propellant or buffer or pH regulator or stabilizer or coating or flavoring agent is formulated as a tablet, coated tablet, capsule, pill, intranasal, lozenges, emulsion, pastilles, suppository, powder, paste or injectable solution.

Embodiment 28 relates the method of embodiment 1, wherein the specific disease is one imposed by an impaired mitobiogenesis, ATP production and an oxidative stress.

Embodiment 29 relates to the method of embodiment 27 or 28, wherein the specific dose is one oral capsule once a day, twice a day or three times a day.

Embodiment 30 relates to the method any one of embodiments 27 to 29, wherein the pharmaceutical composition consists of a Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000 mg, Coenzyme Q10 in the range of 1-3,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Vitamin B Complex contains B1 in the range of 2.4 mg-48 mg, B2 in the range of 3.7 mg-74 mg, B3 in the range of 33 mg -670 mg, B5 in the range of 15 mg-300 mg, B6-3.2 mg-64 mg, B12 in the range of 6 mcg- 120 mcg, Folic acid in the range of 50 mcg-10,000 mcg, Biotin in the range of 110 mcg-2200 mcg, and Taurine in the range of 10-10,000 mg, L-Methionine in the range of 10-20,000 mg, L- Cysteine in the range of 10-20,000 mg.

Embodiment 31 relates to the method of improving bioenergy in a mammal, comprising; administering to the mammal in need thereof a pharmaceutical composition, wherein the pharmaceutical composition is a Magnesium Citrate, Alpha-Ketoglutaric Acid, Niacinamide, L Glutamine, L Citrulline, Sodium Pyruvate, Succinic acid, Taurine, L-Methionine, L- Cysteine or combination thereof, is formulated as a tablet, coated tablet, capsule, pill, intranasal, lozenges, emulsion, pastilles, suppository, powder, paste or injectable solution.

Embodiment 32 relates to the method of embodiment 31, further consisting of a mix containing the Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, L- Cysteine or combination thereof.

Embodiment 33 relates to the method of embodiment 31, further consisting of mix having a Mix G 12 consisting of the Magnesium Citrate, Alpha-Ketoglutaric Acid, Niacinamide, L Glutamine, L Citrulline, Sodium Pyruvate, Succinic acid, Vitamin B Complex, Taurine, L-Methionine, and L- Cysteine.

Embodiment 34 relates to the method of embodiment 31, further comprising; a Vitamin C, Vitamin B complex and Coenzyme Q10.

Embodiment 35 relates to the method of embodiment 31, wherein the pharmaceutical composition comprises of the Magnesium Citrate in the range of 10-30,000 mg, Alpha-Ketoglutaric Acid in the range of 10-30 ,000 mg, Niacinamide in the range of 1-10,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Taurine in the range of 10-10,000 mg, L-Methionine in the range of 10-20,000 mg, L- Cysteine in the range of 10-20,000 mg.

Embodiment 36 relates to the method of embodiment 31, wherein the pharmaceutical composition comprises of the Vitamin C as L- Ascorbic acid in the range of 5-50,000 mg, Coenzyme Q10 in the range of 1-3,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg and Folic acid in the range of 50 mcg-10,000 mcg, and Biotin in the range of 110 mcg-2200 mcg.

Embodiment 37 relates to the method of embodiment 35, further comprising a specific disease, wherein the specific disease is due to one of an impaired mitobiogenesis ATP production and an oxidative stress.

Embodiment 38 relates to the method of of treating the specific disease by administering a pharmaceutical composition to a mammal, comprising; a Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000 mg, Coenzyme Q10 in the range of 1-3,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg, Folic acid in the range of 50 mcg-10,000 mcg, Biotin in the range of 110 mcg-2200 mcg, and Taurine in the range of 10-10,000 mg, L-Methionine in the range of 10-20,000 mg, L- Cysteine in the range of 10-20,000 mg and is formulated as a tablet, coated tablet, capsule, pill, intranasal, lozenges, emulsion, pastilles, suppository, powder, paste or injectable solution.

Embodiment 39 relates to the method of method embodiment 38, wherein the mammal is human. Embodiment 40 relates to the method of embodiment 38, wherein the formulation is at least one of an oral, nasal, topical, rectal, vaginal, aerosol, parenteral administration or a combination thereof.

## Claims

1. A pharmaceutical composition comprising at least one ingredient selected from the group consisting of Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine, L- Cysteine and combination thereof, preferably wherein the pharmaceutical composition comprises Magnesium Citrate, Alpha-Ketoglutaric Acid, Niacinamide, Vitamin C, Coenzyme Q10, L Glutamine, L Citrulline, Sodium Pyruvate, Succinic acid, Vitamin B Complex, Taurine, L-Methionine and L-Cysteine.

2. A pharmaceutical composition of claim 1, wherein the pharmaceutical composition comprises or consists of a Magnesium Citrate in the range of 10-30,000 mg, Alpha-Ketoglutaric Acid in the range of 10-30 ,000 mg, Niacinamide in the range of 1-10,000 mg, Vitamin C as L- Ascorbic acid in the range of 5-50,000 mg, Coenzyme Q10 in the range of 1-3,000 mg, L Glutamine in the range of 10-50,000 mg, L Citrulline in the range of 10-20,000 mg, Sodium Pyruvate in the range of 10-50,000 mg, Succinic acid in the range of 10-30,000 mg, Vitamin B Complex contains B1 in the range of 2.4 mg-48 mg, B2 in the range of 3.7 mg- 74 mg, B3 in the range of 33 mg -670 mg, B5 in the range of 15 mg-300 mg, B6-3.2 mg-64 mg, B12 in the range of 6 mcg- 120 mcg, Folic acid in the range of 50 mcg-10,000 mcg, Biotin in the range of 110 mcg-2200 mcg, and Taurine in the range of 10-10,000 mg, L-Methionine in the range of 10-20,000 mg, L-Cysteine in the range of 10-20,000 mg.

3. The pharmaceutical composition of claim 1 or 2 comprising one or combination of pharmaceutically acceptable carriers or excipient or liquefied propellant or buffer or pH regulator or stabilizer or coating or flavoring agent, wherein the pharmaceutical composition is formulated as a tablet, coated tablet, capsule, pill, intranasal, lozenges, emulsion, pastilles, suppository, powder, paste or injectable solution.

4. A pharmaceutical composition of any one of claims 1 to 3, for use in medicine.

5. A pharmaceutical composition of any one of claims 1 to 3 for use in treating a mammal having a disease which is one imposed by an impaired mitobiogenesis ATP production and an oxidative stress, preferably wherein the disease is selected from the group consisting of arrhythmia, heart failure, cardiovascular conditions, depression, mental decline and dementia.

6. The pharmaceutical composition for use of claim 4 or 5, wherein the pharmaceutical composition is an oral capsule, preferably wherein the oral capsule is to be administered in a ose of one oral capsule once a day, twice a day or three times a day.

7. A pharmaceutical composition of any one of claims 1 to 3 for use in improving bioenergy in a mammal.

8. The pharmaceutical composition for use of claim 7, wherein the mammal suffers from a specific disease, wherein the specific disease is due to one of an impaired mitobiogenesis ATP production and an oxidative stress, preferably wherein the disease is selected from the group consisting of arrhythmia, heart failure, cardiovascular conditions, depression, mental decline and dementia.

9. The pharmaceutical composition for use of claim 7, further comprising a mix containing Magnesium Citrate, Alpha-Ketoglutaric Acid, Niacinamide, Vitamin C, Coenzyme Q10, L Glutamine, L Citrulline, Sodium Pyruvate, Succinic acid, Taurine, L-Methionine, L-Cysteine or combination thereof.

10. The pharmaceutical composition for use of claim 7, further comprising a mix Mix G 12 containing the Magnesium Citrate, Alpha-Ketoglutaric Acid, Niacinamide, L Glutamine, L Citrulline, Sodium Pyruvate, Succinic acid, Vitamin B Complex, Taurine, L-Methionine, and L- Cysteine.

11. The pharmaceutical composition for use of any one of claims 4 to 10, further comprising; a Vitamin C, Vitamin B complex and Coenzyme Q10.

12. The pharmaceutical composition of any one of claims 5 to 11, wherein the mammal is human.
